# EUROPEAN PATENT SPECIFICATION

(11) **EP 1 539 116 B1**
(45) Date of publication and mention of the grant of the patent: **17.02.2016**
(21) Application number: 03788474.9
(22) Date of filing: 14.08.2003
(51) Int. Cl.: A61K 9/00, A61K 9/20

(54) **NON-ANIMAL PRODUCT CONTAINING VETERINARY FORMULATIONS**
Veterinärmedizinische Formulierungen frei von tierischen Produkten
FORMULATIONS VETERINAIRES NE CONTENANT PAS DE PRODUITS D'ORIGINE ANIMALE

(30) Priority: 16.08.2002 US 222559
(43) Date of publication of application: 15.06.2005
(73) Proprietor: Merial, Inc., Duluth, GA 30096 (US)
(72) Inventor: CLEVERLY, Douglas, Milltown, NJ 08850 (US); HAGENBUCH, Michelle, Greenbrook, NJ 08812-2217 (US); CHEN, Jun, Robbinsville, NJ 08691 (US); AZAD, Abul, South Brunswick, NJ 08810 (US); MUHITCH, James, Monmounth Junction, NJ 08852 (US)
(74) Representative: D Young & Co LLP
(86) International application number: PCT/US2003/025448
(87) International publication number: WO 2004/016252

(56) References cited:
- EP-A2- 0 366 101
- EP-A2- 0 717 993
- EP-A2- 1 247 456
- WO-A1-99/17748
- WO-A1-2004/014143
- GB-A- 1 261 299
- US-A- 5 180 720
- US-A1- 2002 028 780
- US-A1- 2003 064 941

## Description

### BACKGROUND OF THE INVENTION

### Field of the Invention

This invention provides for improved oral veterinary formulations, as defined by the claims which do not contain animal products or flavors derived from animal sources, which are palatable to the animal because of their good organoleptic properties, as well as a method to improve the palatability of oral veterinary formulations, without resorting to the use of animal products or flavors derived from animal products. This disclosure further provides for improved chewable veterinary formulations or tablets, which do not contain animal products or flavors derived from animal sources and possess good consistency and acceptablity by the animal, as well as an improved process to prepare chewable veterinary formulations by avoiding a drying step.

### Description of the Related Art

Therapeutic agents are administered to animals by a variety of routes. These routes include, for example, oral ingestion, topical application or parental administration. The particular route selected by the practitioner depends upon factors such as the physiochemical properties of the pharmaceutical or therapeutic agent, the condition of the host, and economics.

For example, one method of formulating a therapeutic agent for oral, topical, dermal or subdermal administration is to formulate the therapeutic agent as a paste or as an injectable formulation and reference is made to US application Ser. No. 09/504,741, filed February 16, 2000, now pending, entitled **IMPROVED PASTE FORMULATIONS** or to Ser. No. 09/346,905, filed July 2, 1999, now pending; Ser. No. 09/112,690, filed July 9, 1999, now allowed; and Ser. No. 09/15,277, filed September 14, 1998, now pending, entitled **LONG ACTING INJECTIBLE FORMULATIONS CONTAINING HYDROGENATED CASTOR OIL**

Other methods include placing the therapeutic a gent in a solid o liquid matrix for oral delivery. These methods include chewable drug-delivery formulations. The problem associated with oral formulations is that the therapeutic agent often provides an unpleasant taste, aroma, or mouth feel to the formulation, which cause, especially in the situation with animals, the oral formulation to be rejected by the patient. See, e.g., U.S. Patent 5,380,535 to Geyer et al.*,* which provides for a lipid based, chewable formulations for oral delivery of therapeutic agents, such as aspirin, ibuprofen or erthromycin, which are unpaletable to humans; U.S. Patent 5,894,029 to Brown et al.*,* which provides for dried puff pet foods comprising farnaceious materials, proteinaceous materials, such as meats or vegetable protein sources, and optionally medicaments or vitamins; or U.S. Patent 5,637,313 to Chau et al.*,* which describes chewable dosage forms comprising a water soluble matrix comprising hydrogenated starch hydrolystate bulking agent and a water insoluble bulking agent.

WO99/17748 discloses granulated taste masked chewable formulations comprising ibuprofen as active ingredient in the absence of animal derived flavours.

Traditionally, in veterinary formulations, palatability had been achieved by the inclusion of animal byproducts or flavors derived from animal sources into the formulation. For example, it is customary to include attracts, such as chicken powder, liver powder, beef, ham, fish, or rawhide-derived products in dog chews to make the chew palatable to the dog. See, e.g., U.S. Patent 6,086,940; U.S. Patent 6,093,441; U.S. Patent 6,159,516; U.S. Patent 6,110,521; U.S. Patent 5,827,565; U.S. Patent 6,093,427, all to Axelrod *et al.* However, the use of animal products or byproducts or flavors derived from animal sources have recently fallen into disfavor because of the possibility of chemical or biological contamination, which lead to toxicity or diseases such as bovine spongiform encephalopathy. Hence, there is a need for oral veterinary formulations that do not contain animal products, byproducts, or flavors derived from animal sources while still exhibiting good organoleptic properties. While non-animal derived products such as valerian plants are know as scent attractants in food products or pet toys (U.S. Patent 5,785,382 to Childers-Zadah) or animal chews that contain fruit flavors as the attractant (see, U.S. Patents 6,274,182; 6,200,616 and 6,126,978 to Axelrod et al.), these patents do not describe using valerian plants or fruit flavors in oral formulations in which the pharmaceutical agents needs to be masked.

**SUMMARY OF THE INVENTION** The present invention which is defined by the claims provides for improved oral veterinary formulations, which do not contain animal products or flavors derived from animal sources, that exhibit organoleptic properties that the animal finds appealing. This disclosure further provides for improved chewable veterinary formulations or which do not contain animal products or flavors derived from animal sources and possess good consistency and acceptablity by the animal, as well as an improved process to prepare chewable veterinary formulations. The present disclosure further provides for a manufacturing process for preparing the inventive chewable veterinary formulations which is a simple, fast and economical process that avoids a drying step, which is customary when animal product or flavors derived from animal sources are employed.

These and other embodiments are disclosed or are obvious, from and encompassed by, the following Detailed Description.

### DETAILED DESCRIPTION

The present disclosure provides for a chewable veterinary formulation, which does not contain animal products, which comprises:
- effective amount of at least one pharmaceutical agent;
- at least one filler;
- at least one disintegrant;
- at least one non-animal product containing flavor or flavor derived from a non-animal source;
- at least one binder;
- at least one humectant;
- at least one granulating solvent; and
- optionally, at least one antioxidant, at least one buffering agent, at least one preservative, or at least one colorant;
or, preferably, a chewable veterinary formulation, which does not contain animal products, which comprises:
-effective amount of a pharmaceutical agent;
-a filler selected from the group consisting of soy protein, corn cob, or corn glutton meal;
-disintegrant;
-a non-animal product containing flavor or a flavor derived from non-animal source which is a hickory smoke flavor;
-a binder;
-humectant;
-granulating solvent; and
-optionally, an antioxidant, a buffering agent, preservative, or a colorant.
Further, the present disclosure provides for a method for enhancing the palatability of an oral veterinary formulation, which does not contain animal products or flavors derived from animal sources which comprises adding a hickory smoke flavor, which optionally further comprises carmel, to the oral veterinary formulation.

This disclosure further provides for a process for preparing a chewable veterinary formulation which does not contain animal products, which comprises the step of:
(a) blending the pharmaceutical agent, binder, disintegrant, and non-animal product containing flavor or flavor derived from a non-animal source;
(b) adding the water and the humectant to the mixture from step (a) and mixing the mixture; and
(c) without drying, extruding the mixture.

Most preferred are chewable veterinary formulations, which do not contain animal products, which comprise:
- an effective amount of a pharmaceutical agent selected from the group consisting of avermectins, milbemycins, nodulisporic acid and its derivatives, estrogens, progestins, androgens, substituted pyridyl methyl derivatives, phenylpyrazols, COX-2 inhibitors, anthelmintic agents and a proton pump inhibitors;
- about 20 to about 60% of a filler selected from the group consisting of soy protein, corn cob, or corn glutton meal;
- about 1 to about 20% of a disintegrant;
- about 0.1 to about 20% of a non-animal product containing flavor; or a flavor derived from a non-animal source
- about 0.5 to 10% a binder;
- about 5 to about 20% of a humectant; and
- about 5 to about 20% granulating solvent,
based upon total weight of formulation. Especially preferred are chewable veterinary formulations, which do not contain animal products which comprise:
- an effective amount of a pharmaceutical agent;
- about 20 to about 60% of a filler selected from the group consisting of soy protein, corn cob, or corn gluten meal;
- about 1 to about 20% of a disintegrant;
- about 0.1 to about 20% of the non-animal product containing flavor or flavor derived from a non-animal source is a hickory barbecue flavor;
- about 0.5 to 10% a binder;
- about 5 to about 20% of a humectant; and
- about 5 to about 20% granulating solvent,
and, optionally
- about 0.05% to about 1.0% of an antioxidant,
- about 0.05 to about 1.0% of a preservative, and
- about 0.001 to about 10% of a colorant,
based upon total weight of formulation. The formulations wherein the pharmaceutical agent is fipronil or a COX-2 inhibitor are especially preferred.

Also preferred are chewable veterinary formulations which comprise a combination of at least two pharmaceutically active ingredients. Especially preferred are chewable veterinary formulations wherein the pharmaceutically active ingredients are praziquantel and eprinomectin.

Another preferred embodiment is a tablet, which does not contain animal products, which comprises:
- an effective amount of at least one pharmaceutical agent
- at least one filler;
- at least one non-animal product containing flavor or flavor derived from a non-animal source;
- at least one lubricant;
- at least one flow aid; and
- optionally, at least one antioxidant, at least one pH modifier, at least one binder, at least one disintegrant, at least one surfactant, at least one preservative, and at least one colorant, and is optionally coated with at least one coating.

The pharmaceutical or therapeutic agents which are used in the disclosed formulations are those which are known to the practitioner as agents which may be formulated as oral formulations. Classes of pharmaceutical agents contemplated by the inventive formulations include insecticides, acaricides, parasiticides, growth enhancers, oil-soluble, nonsteroidal anti-inflammatory drugs (NSAIDS), proton pump inhibitors and antibacterial compounds. Specific classes of compounds which fall within these classes include, for example, avermectins, milbemycins, nodulisporic acid and its derivatives, estrogens, progestins, androgens, substituted pyridylmethyl derivatives, phenylpyrazoles, COX-2 inhibitors, 2-(2-benzimidazolyl)-pyrimidines derivatives, macrolide antibiotics 2-acyl-4-oxo-pyrazino-isoquinoline derivatives, such as praziquantel or 1,4.5,6-tetrahydro-2-[2-substituted]vinyl pyrimidines and 2-[(2-substituted)vinyl]-2-imidazolines such as pyrantel (see U.S. Patent 3,502,661).

The avermectin and milbemycin series of compounds are potent anthelmintic and antiparasitic agents against a wide range of internal and external parasites. The compounds which belong to this series are either natural products or are semi-synthetic derivatives thereof. The structure of these two series of compounds are closely related and they both share a complex 16-membered macrocyclic lactone ring; however, the milbemycin do not contain the aglycone substituent in the 13-position of the lactone ring. The natural product avermectins are disclosed in U.S. Patent 4,310,519 to Albers-Schonberg, et al.*,* and the 22, 23-dihydro avermectin compounds are disclosed in Chabala, et al., U.S. Patent 4,199,569. For a general discussion of avermectins, which include a discussion of their uses in humans and animals, see "Ivermectin and Abamectin," W.C. Campbell, ed., Springer-Verlag, New York (1989). Naturally occurring milbemycins are described in Aoki et al., U.S. Patent 3,950,360 as well as in the various references cited in "The Merck Index" 12th ed., S. Budavari, Ed., Merck & Co., Inc. Whitehouse Station, New Jersey (1996). Semisynthetic derivatives of these classes of compounds are well known in the art and are described, for example, in U.S. Patent 5,077,308, U.S. Patent 4,859,657, U.S. Patent 4,963,582, U.S. Patent 4,855,317, U.S. Patent 4,871,719, U.S. Patent 4,874,749, U.S. Patent 4,427,663, U.S. Patent 4,310,519, U.S. Patent 4,199,569, U.S. Patent 5,055,596, U.S. Patent 4,973,711, U.S. Patent 4,978,677, and U.S. Patent 4,920,148.

Avermectins and milbemycins share the same common 16-membered macrocyclic lactone ring; however, milbemycins do not possess the disaccharide substituent on the 13-position of the lactone ring.

While many avermectin compounds are known in the art, a representative 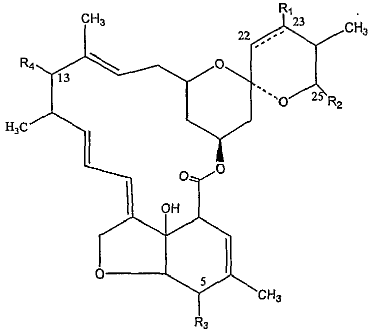 structure of the class of compounds is as follows:
where the broken line indicates a single or a double bond at the 22,23-positions;
R₁ is hydrogen or hydroxy provided that R₁ is present only when the broken line indicates a single bond;
R₂ is alkyl of from 1 to 6 carbon atoms or alkenyl of from 3 to 6 carbon atoms or cycloalkyl of from 3 to 8 carbon atoms;
R₃ is hydroxy, methoxy or = NOR₅ where R₅ is hydrogen or lower alkyl; and
R₄ is hydrogen, hydroxy or 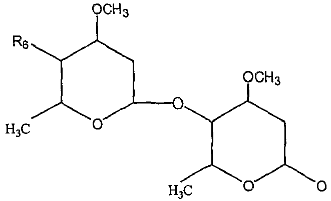 where R₆ is hydroxy, amino, mono-or di-lower alkylamino or lower alkanoylamino.

The preferred compounds are avermectin B1a/B1b (abamectin), 22,23-dihydro avermectin B1a/B1b (ivermectin) and the 4"-acetylamino-5-ketoximino derivative of avermectin B1a/B1b. Both abamectin and ivermectin are approved as broad spectrum antiparasitic agents.

The structures of abamectin and ivermectin are as follows: 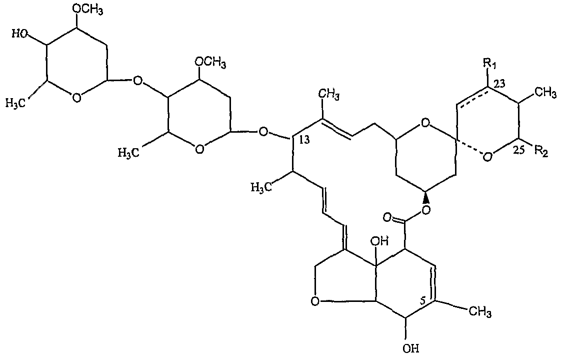 wherein for abamectin the broken line represents a double bond and R₁ is not present and for ivermectin the double bond represents a single bond and R₁ is hydrogen; and
R₂ is isopropyl or sec-butyl.

The 4"-acetyl amino-5-ketoximino derivatives of avermectin B1a/B1b has the following structural formula: 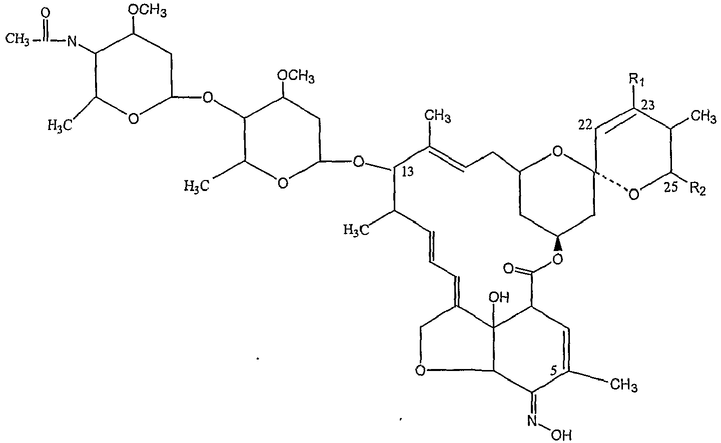 where R₂ is isopropyl or sec-butyl.

The avermectin products are generally prepared as a mixture of at least 80% of the compound where R₂ is sec-butyl and no more than 20% of the compound where R₂ is isopropyl.

Other preferred avennectins, include ememectin, eprinomectin and doramectin. D oramectin is disclosed in U.S. Patent 5,089,490 and E P 214 738. This compound has the following structure: 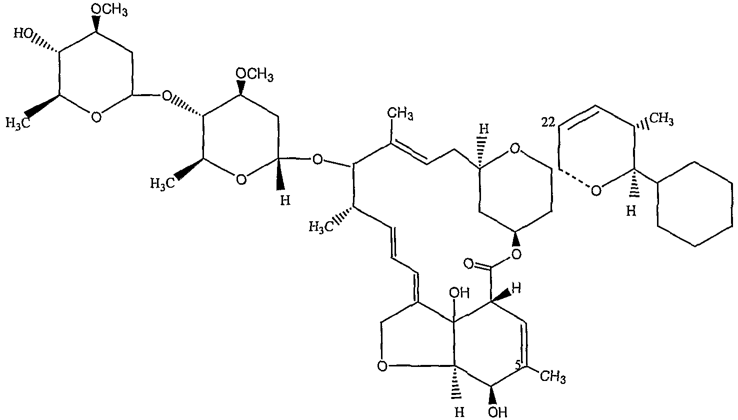 In the present formulations, ivermectin and eprinomectin are especially preferred.

A representative structure for a milbemycin is that for milbemycin α₁:

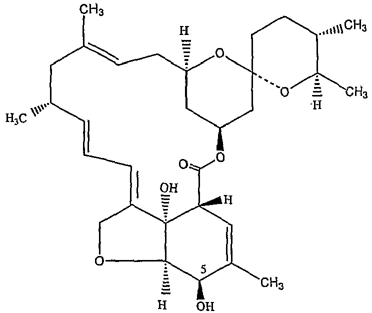

An e specially preferred m ilbemycin is moxidectin, whose structure is as follows: 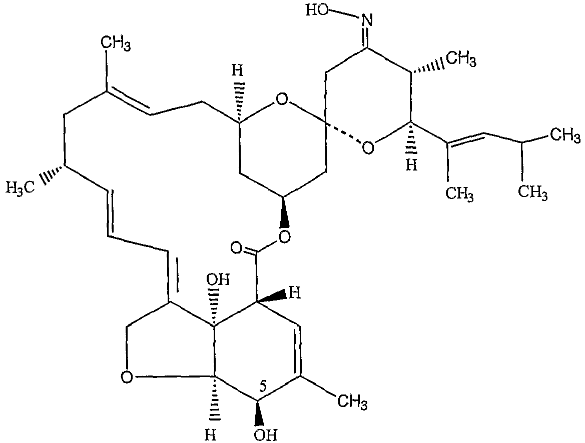 The compound is disclosed in U.S. Patent No. 5,089,490.

The monosaccharide avermectin derivatives are also preferred especially where an oxime substitution is present on the 5-position of the lactone ring. Such compounds are described, for example, in EP 667,054. Selamectin is an especially preferred compound of this class of derivatives.

Nodulisporic acid and its derivatives are a class of acaricidal, antiparasitic, insecticidal and anthelmintic agents well known to a practitioner of the art. These compounds are used to treat or prevent infections in humans and animals. These compounds are described, for example, in U.S. Patent 5,399,582 and WO 96/29073. Additionally, the compounds can be administered in combination with other insecticides, parasiticides, and acaricides. Such combinations include anthelmintic agents, such as those discussed above which include ivermectin, avermectin, and emamectin, as well as other agents such as thiabendazole, febantel or morantel; phenylpyrazoles such as fipronil; and insect growth regulators such as lufenuron. Such combinations are also contemplated in the present disclosure

Generally, all classes of insecticides are provided for in this disclosure. One example of this class include substituted pyridylmethyl derivatives such as imidacloprid. Agents of this class are described, for example, in U.S. Patent 4,742,060 or in EP 892,060. It would be well within the skill level of the practitioner to decide which individual compound can be used in the inventive formulation to treat a particular infection of an insect.

Phenylpyrazoles are another class of insecticides which possess excellent insecticidal activity against all insect pests including blood-sucking pests such as ticks, fleas etc., which are parasites on animals. This class of agents kills insects by acting on the gamma-butyric acid receptor of invertebrates. Such agents are described, for example, in U.S. Patent No. 5,567,429, U.S. Patent No. 5,122,530, U.S. Patent 5,232,940 and EP 295,117. An especially preferred phenylpyrazole is fipronil, whose chemical name is 5-amino-3-cyano-1-(2,6-dichloro-4-trifluoromethylphenyl)-4-trifluoromethylpyrazole. Fipronil is well known in the art as a flea and tick agent. It would be well within the skill level of the practitioner to decide which individual compounds can be used in the inventive formulations. Other preferred phenyl pyrazoles include the following compounds:

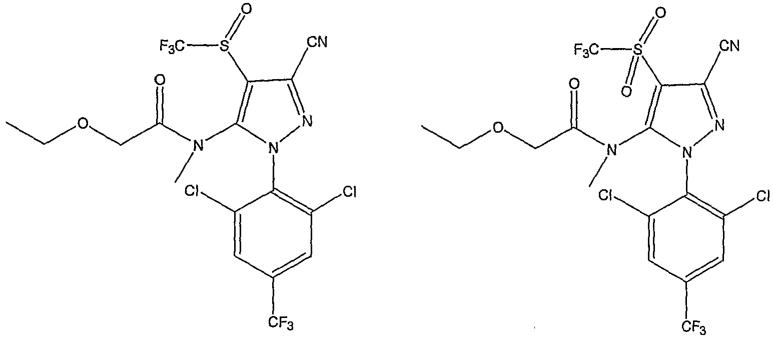

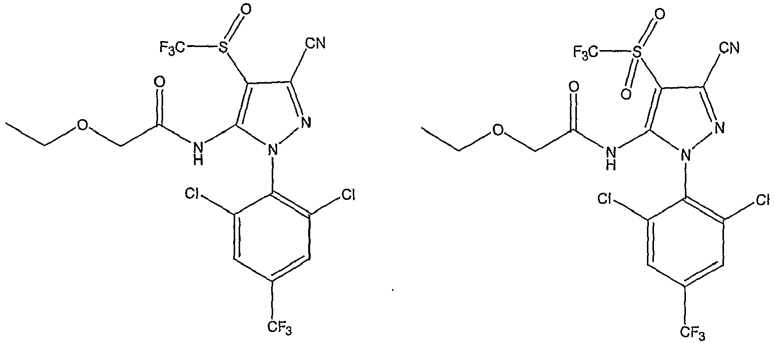

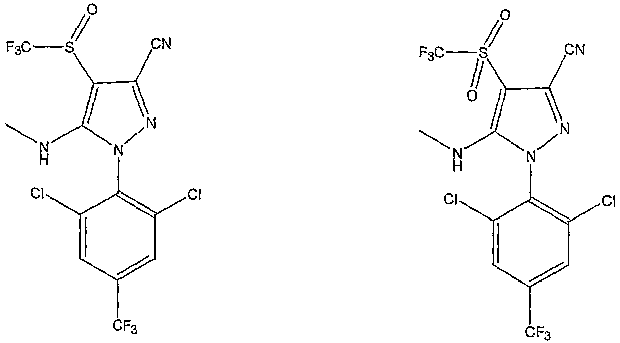

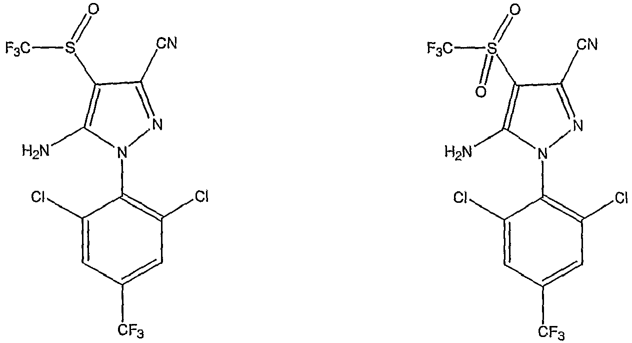

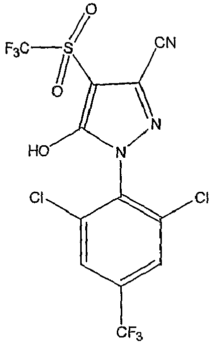

Especially preferred phenylpyrazoles in addition to fipronil include fipronil thio 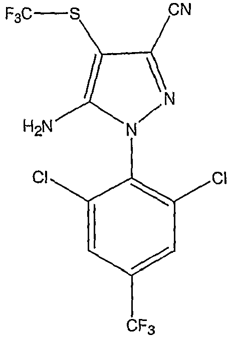 and fipronil sulfone

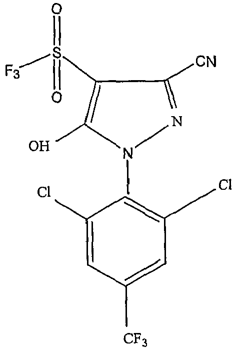

Insect growth regulators are another class of insecticides or acaricides, which are also provided for in the inventive formulations. Compounds belonging to this group are well known to the practitioner and represent a wide range of different chemical classes. These compounds all act by interfering with the development or growth of the insect pests. Insect growth regulators are described, for example, in U.S. Patent 3,748,356; U.S. Patent 3,818,047; U.S. Patent 4,225,598; U.S. Patent 4,798,837; and U.S. Patent 4,751,225, as well as in EP 179,022 or U.K. 2,140,010. Especially preferred insect growth regulators include diflubenzuron, lufenuron, methoprene, phenoxycarb, pyriproxyfen, and cyromazine. Again, it would be well within the skill level of the practitioner to decide which individual compounds can be used in the inventive formulation.

Estrogens, progestins, and androgens refers to classes of chemical compounds which are also well known to a practitioner in this art and used, for example, to regulate fertility in humans and animals. In fact, estrogens and progestins are among the most widely prescribed drugs and are used, for example, alone or in combination for contraception or hormone replacement therapy in post menopausal women. Estrogens and progestins occur naturally or are prepared synthetically. This class of compounds also includes estrogens or progesterone receptor antagonists. Antiestrogens, such as tamoxifen and clomiphene, are used to treat breast cancer and infertility. Antiprogestives are used as contraceptives and anticancer drugs, as well as to induce labor or terminate a pregnancy.

The androgens and antiandrogens structurally related to the estrogens and progestins as they are also biosynthesized from cholesterol. These compounds are based on testosterone. Androgens are used for hypogonadism and promote muscle development. Antiandrogens are used, for example, in the management of hyperplasia and carcinoma of the prostate, acne, and male pattern baldness as well as in the inhibition of the sex drive in men who are sex offenders. Estrogen, progestins, and androgens are described, for example, in "Goodman & Gilman's The Pharmacological Basis of Therapeutics," 9th ed., J.G. Handman and L. Elimbird, eds., Ch. 57 to 60, pp. 1411-1485, McGraw Hill, New York (1996) or in "Principles of Medicinal Chemistry," 2nd ed., W.O. Foye, ed., Ch. 21, pp. 495-559, Lea & Febiger, Philadelphia (1981).

Estrogens, progestins and androgens are also used in animal husbandry as growth promoters for food animals. It is known in the art that compounds of these classes act as growth-promoting steroids in animals such as cattle, sheep, pigs, fowl, deer, rabbits, etc. Delivery systems to promote the growth of animals are described, for example, in U.S. Patent 5,401,507, U.S. Patent 5,288,469, U.S. Patent 4,758,435, U.S. Patent 4,686,092, U.S. Patent 5,072,716 and U.S. Patent 5,419,910.

Specific estrogen, progestin and androgen compounds are well known to the practitioner. Especially preferred compounds belonging to this class include progesterone, estradiol benzoate and trenbolone acetate.

NSAIDS are well known in the art. The classes of compounds which belong to this group include salicylic acid derivatives, para-aminophenol derivatives, indole and indene acetic acids, heteroaryl acetic acids, arylpropionic acids, anthranilic acids (fenamates), enolic acids, and alkanones. NSAIDS exert their activity by interfering with prostaglandin biosynthesis by irreversibly or reversibly inhibiting cycloxygenase. Compounds of this group possess analgesic, antipyretic and nonsteroidal anti-inflammatory properties. Compounds belonging to these classes are described, for example, in Chapter 27 of Goodman and Gilman on pages 617 to 658 or in Ch. 22 of Foye on pages 561 to 590 as well as in U.S. Patents 3,896,145; U.S. Patent 3,337,570; U.S. Patent 3,904,682; U.S. Patent 4,009,197; U.S. Patent 4,223,299; and U.S. Patent 2,562,830, as well as the specific agents listed in The Merck Index. This invention contemplates those compounds that are oil-soluble.

Oil-soluble NSAIDS are also well known to the practitioner. Classes of NSAIDS which are preferred are indole and indecene acetic acids and heteroaryl acetic acids. Especially preferred compounds include indomethacin, ketorolac, caprofen, flunixin, ketoprofen, meloxicam, naproxen, and phenylbutazone.

COX-2 inhibitors are an especially preferred class of NASIDS. As with other NASIDS, COX-2 inhibitors are effective in treating cycloxygenase mediated diseases such as inflammation, analgesia and fever. These compounds are especially effective in treating cancer, rheumatoid arthritis and osteoarthritis. These compounds have the advantage of not affecting the integrity of the gastrointestinal tract and the renal blood flow. Examples of these compounds include (methylsulfonyl)phenyl-2-5(H)-furanone derivatives. These derivatives are described, for example, in copending application USSN 09/097,537, now allowed, which in turn is a CIP of application USSN 08/728,512, filed on October 9, 1996, which in turn is based upon provisional applications nos. 60/005,371 and 06/011,673. Especially preferred COX-2 inhibitors include 3-(cyclopropylmethoxy)-5,5-dimethyl-4-(4-methylsulfonyl)phenyl)-5H-furan-2-one or 3-(cyclopropylethoxy)-5,5-dimethyl-4-(4-methylsulfonyl)phenyl)-5H-furan-2-one or pharmaceutically acceptable salts or hydrates of these compounds. An especially preferred COX-2 inhibitor is polymorphic form B of 3-(cyclopropylmethoxy)-4-[4(methylsulfonyl)phenyl]-5,5-dimethyl-5H-furan-2-one.

Compounds which inhibit gastric acid secretion in the stomach or act as proton pump inhibitors are well known to the practitioner and are also provided for in the present invention. These compounds include 2-(2-benzimidazolyl-pyridines) and their salts. Such compounds are described, for example in EP 005 129, U.S. Patent 4,255,431 as well as in U.S. Patent 5,629,305. These compounds are also known to treat Helicobacter infections, U.S. Patent 5,093,342, and to act as synergists when combined with an acid degradable antibiotic, see e.g. U.S. 5,629,305. These synergistic combinations may also be formulated in the pastes of the present invention. Omeprazole or its salts is an especially preferred compound.

Macrolide antibiotics are also preferred therapeutic agents. Macrolides as a class include the erythromycin and its derivative as well as other derivatives such as the azalides. Erythromycin (MW 733.94 daltons) is the common name for a macrolide antibiotic produced by the growth of a strain of Streptomyces erythreous. It is a mixture of three erythromycins, A, B and C consisting largely of erythromycin A which is represented by the formula:

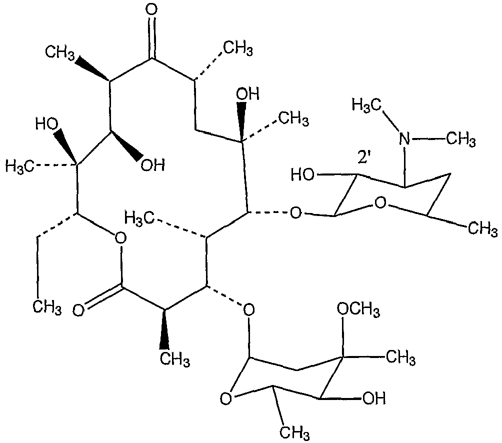

Its chemical name is (3R^{*},4S^{*},5S^{*},6R^{*},7R^{*},9R^{*},11R^{*},12R^{*}, 13S^{*},14R^{*})-4-[(2,6-dideoxy-3-C-methyl-3-O-methyl-α-L-ribo-hexopyranosyl)-oxy]-14-ethyl-7,12,13-trihydroxy-3,5,7,9,11,13-hexamethyl-6[[3,4,6-trideoxy-3-(dimethylamino)-β-D-xylohexapyranosyl]oxy]oxacyclotetradecane-2,10-dione, (C₃₇H₆₇NO₁₃).

Erythromycin has a broad and essentially bacteriostatic action against many Gram-positive and some Gram-negative bacteria as well as other organisms including mycoplasmas, spirochetes, chlamydiae and rickettsiae. In humans, it finds usefulness in the treatment of a wide variety of infections. It finds wide application in veterinary practice in the treatment of infectious diseases such as pneumonias, mastitis, metritis, rhinitis, and bronchitis in, for example, cattle, swine and sheep.

Other derivatives of erythromycins include carbomycin, clarithromycin, josamycin, leucomycins, midecamycins, mikamycin, miokamycin, oleandomycin, pristinamycin, rokitamycin, rosaramicin, roxithromycin, spiramycin, tylosin, troleandomycin, and virginiamycin. As with the erythromycins, many of these derivatives exist as component mixtures. For example, carbomycin is a mixture of carbomycin A and carbomycin B. Leucomycin exists as a mixture of components A₁, A₂, A₃, A₉, B₁-B₄, U and V in various proportions. Component A₃ is also known as josamycin and leucomycin V is also known as miokomycin. The major components of the midecamycins is midecamycin A and the minor components are midecamycins A₂, A₃ and A₄. Likewise, mikamycin is a mixture of several components, mikamycin A and B. Mikamycin A is also known as virginiamycin M₁. Pristinamycin is composed of pristinamycins I_{A}, I_{B}, and I_{C}, which are identical to virginiamycins B₂, B₁₃ and B₂ respectively, and pristinamycin II_{A} and II_{B}, which are identical to virginiamycin M₁ and 26,27-dihydrovirginiamycin M₁. Spiramycin consists of three components, spiromycin I, II, and III. Virginiamycin is composed of virginiamycin S₁ and virginiamycin M₁. All these components may be used in this invention. Sources of these macrolides are well known to the practitioner and are described in the literature in references such as "The Merck Index," 12th ed., S. Budarari, ed., Merck & Co., Inc., Whitehouse Station, NJ (1996).

Azalides are semisynthetic macrolides antibiotics related to erythromycin A and exhibit similar solubility characteristics. This class includes compounds of the general structure 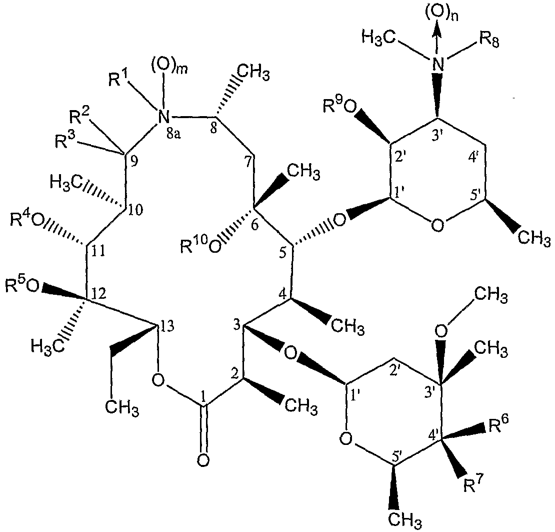 and the pharmaceutically acceptable salts and esters thereof, and the pharmaceutically acceptable metal complexes thereof, wherein
R¹ is hydrogen;
   hydroxy;
   C₁₋₄ alkoxy;
   formyl;
   C₁₋₁₀ alkylcarbonyl, C₁₋₁₀ alkoxycarbonyl, aryloxycarbonyl, C₁₋₁₀ aralkoxycarbonyl, C₁₋₁₀ alkylsulfonyl, or arylsulfonyl wherein said C₁₋₁₀ alkyl group or aryl group is unsubstituted or substituted by 1-3 halo (F, Cl, Br), hydroxy, amino, C₁₋₅ acylamino or C₁₋₄ alkyl groups; or unsubstituted or substituted C₁₋₁₀ alkyl, C₂₋₁₀ alkenyl or C₂₋₁₀ alkynyl wherein said substituents are independently 1-3 of
   (a) aryl or heteroaryl optionally substituted by 1-3 halo (F, Cl, Br, I), C₁₋₄ alkyl, C₁₋₃ alkoxy, amino, C₁₋₄ alkylamino, di(C₁₋₄ alkyl) amino or hydroxy,
   (b) heterocyclyl optionally substituted by hydroxy, amino, C₁₋₄ alkylamino, di(C₁₋₄ alkyl)amino, C₁₋₄ alkylcarbonyloxy or C₁₋₄ alkylcarbonylamino,
   (c) h alo (F, Cl, Br or I),
   (d) hydroxy optionally acylated by a group 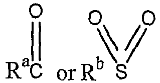 wherein
      R^{a} is hydrogen, C₁₋₆ alkyl, aryl, heteroaryl, aralkyl, or heteroaralkyl and
      R^{b} is C₁₋₆ alkyl or aryl,
   (e) C₁₋₁₀ alkoxy,
   (f) aryloxy or heterocarboxy optionally substituted by 1-3 halo, hydroxy, amino or C₁₋₄ alkyl groups,
   (g) amino or C₁₋₁₀ alkylamino optionally acylated by a group 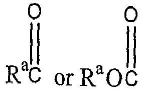 or R^{b}SO₂, wherein
   R^{a} and R^{b} are defined as above,
   (g) di(C₁₋₁₀ alkyl)amino,
   (h) arylamino, heteroarylamino, aralkylamino or heteroarylakylamino wherein said aryl or heteroaryl group is optionally substituted by 1-3 halo, hydroxy, amino or C₁₋₄ alkyl groups,
   (i) mercapto,
   (j) C₁₋₁₀ alkylthio, alkylsulfinyl or alkylsulfonyl, arylthio, arylsulfinyl or arylsulfonyl wherein said aryl group is optionally substituted by 1-3 halo, hydroxy, amino or C₁₋₄ alkyl groups,
   (k) formyl,
   (l) C₁₋₁₀ alkylcarbonyl,
   (m) arylcarbonyl, heteroarylcarbonyl, aralkylcarbonyl or heteroarylalkylcarbonyl wherein said aryl or heteroaryl group is optionally substituted by 1-3 halo, hydroxy, amino or C₁₋₄ alkyl groups,
   (n) carboxy,
   (o) C₁₋₁₀ alkoxycarbonyl,
   (p) aryloxycarbonyl, heteroaryloxycarbonyl, aralkoxycarbonyl or heteroarylalkoxycarbonyl wherein s aid aryl or heteroaryl group is optionally substituted by 1-3 halo, hydroxy, amino or C₁₋₄ alkyl groups,
   (q) carbamoyl or sulfamoyl wherein the N-atom is optionally substituted by 1-2 C₁₋₆ alkyl groups or by a C₄₋₆ alkylene chain,
   (r) cyano,
   (s) isonitrilo,
   (t) nitro,
   (u) azido,
   (v) iminomethyl optionally substituted on nitrogen or carbon with C₁₋₁₀ alkyl,
   (w) oxo, or
   (x) thiano;
wherein said alkyl chain, if more than two carbons in length, can be optionally interrupted by 1-2 oxa, thia or aza (-NR-wherein R is hydrogen or C₁₋₃ alkyl) groups.
R¹⁰ is hydrogen or
R¹ and R¹⁰ together are C₁-C₃ alkylene optionally substituted by an oxo group;
R¹ and R⁴ together are C₁-C₃ alkylene optionally substituted by an oxo group
R² and R³ are hydrogen, C₁₋₁₀ alkyl, aryl
R² and R³ together are oxo and thiano;
R⁴ and R⁵ are independently hydrogen and alkylcarbonyl;
R⁴ and R⁵ are together carbonyl;
R⁶ and R⁷ are both hydrogen or one of R⁶ and R⁷ is hydrogen and the other is hydroxy, an acyloxy derivative taken from the group consisting of formyloxy,
   C₁₋₁₀ alkylcarbonyloxy, arylcarbonyloxy and aralkylcarbonyloxy, or
   -NHR¹² wherein R¹² is hydrogen, arylsulfonyl or heteroarylsulfonyl optionally substituted by 1-3 halo or C₁₋₃ alkyl groups, alkylsulfonyl, or 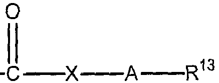 where
   X is a connecting bond, O or NH,
   A is a connecting bond or C₁-C₃ alkylene
   R¹³ is hydrogen, C₁-C₁₀ alkyl, aryl, aralkyl, heteroaryl, heterocyclyl, or C₃-C₇ cycloalkyl, any of which R¹³ groups other than hydrogen c an be substituted by one or more of halogen, hydroxyl, C₁-C₃ alkoxy, cyano, isonitrilo, nitro, amino, mono- or di-(C₁-C₃) alkylamino, mercapto, C₁-C₃ alkylthio, C₁-C₃ alkylsulfinyl, C₁-C₃ alkylsulfonyl, arylthio, arylsulfinyl, sulfamoyl, arylsulfonyl, carboxy, carbamoyl, C₁-C₃ alkylcarbonyl, or C₁-C₃ alkoxycarbonyl;
   R⁶ and R⁷ are together oxo, hydroxyimino, alkoxyimino, aralkoxyimino or aminoimino;
   R⁸ is methyl, aralkoxycarbonyl, and arylsulfonyl;
   R⁹ is hydrogen, formyl, C₁₋₁₀ alkylcarbonyl, C₁₋₁₀ alkoxycarbonyl, and arylalkoxycarbonyl;
   m and n are independently integers of zero or one; and said metal complex is taken from the group consisting of copper, zinc, cobalt, nickel and cadmium.

These compounds are disclosed in EP 568 699.

Azalides as a class of components is well-known in the art and further derivatives are described, for example, in U.S. Patent Nos. 5,869,629; 5,629,296; 5,434,140; 5,332,807; U.S. 5,250,518; 5,215,890; and 5,210,235.

Particularly preferred is azithromycin. The structure of azithromycin is

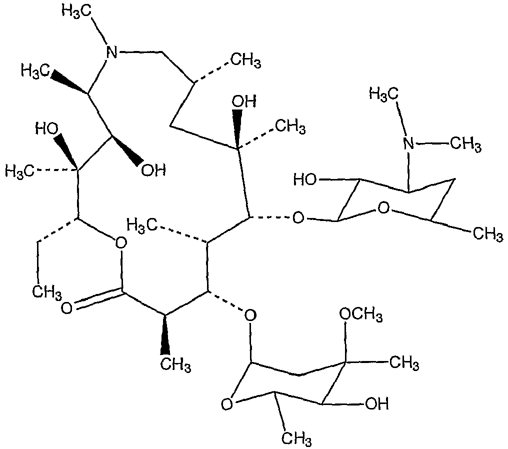

Compounds termed herein formula I and formula II have the following structures: 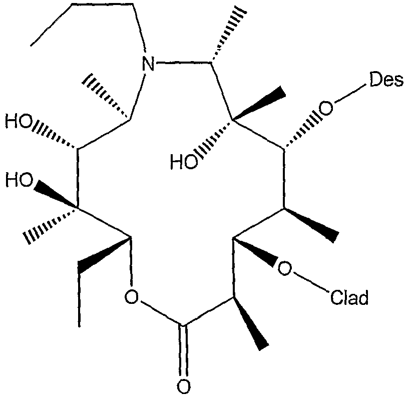 wherein Des is desosomine and Clad is cladinose (formula I) and 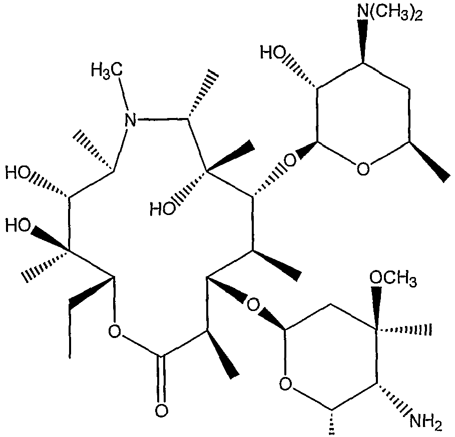 (formula II). The compound of formula II are also known as 8a-azalide. These compounds are disclosed in EP 508 699. The corresponding basic and acid addition salts and ester derivatives of the macrolides, including the azalides compounds, are also contemplated. These salts are formed from the corresponding organic or inorganic acids or bases. These derivatives include the customary hydrochloride and phosphate salts as well as the acetate, propionate and butyrate esters. These derivatives may have different names. For example, the phosphate salt of oleandomycin is matromycin and the triacetyl derivative is troleandomycin. Rokitamycin is leucomycin V 4-B-butanoate, 3B-propionate.

Other pharmaceutical or therapeutic agents are those known in the art to treat parasitic infection caused by nematodes and trematodes. In order to treat cestode (and trematode) infections in warm-blooded animals, it is know, to administer 2-acyl-4-oxo-pyrazino-isoquinoline derivatives to the animal (see, e.g., U.S. 4,001,441, herein A compound of this class that is often used to treat cestode and nematode infections is praziquantel, which has the following structure:

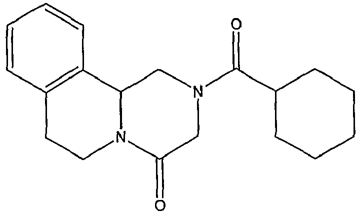

Often it is beneficial to administer a formulation that contains a combination of two or more anthelmintics, which possess different activity, in order to obtain a composition with a broad spectrum of activity. For example, avermectin are ineffective against cestodes, such as tapeworms, and thus are ineffective against an infestation caused by roundworms and tapeworms. Further, the combination allows the user to administer one formulation instead of two or more different formulations to the animal. Formulations which administer a combination of two or more anthelmintics are know in the art. These formulations may be in the form of solutions, suspensions, pastes, drenches or pour-on formulations (see, e.g., U.S. Patent 6,165,987 to Harvey, U.S. Patent 6,340,672 to Mihalik or copending application USSN 10/177,822 entitled **Anthelmintic Oral Homogeneous Veterinary Pastes,** filed on June 21, 2002). For example, U.S. Patent 4,468,390 to Kitano and U.S. Patent 5,824,653 to Beuvry et al. describe anthelmintic compositions for treating nematode and cestode infections in animals, such as horses, that comprise an avermectin or a milbemycin and an isoquinoline compounds, such as praziquantel, to the animal. In these formulations, the avermectin or milbemycin compound and the isoquinoline compound. Similarly, U.S. Patent 6,207,179 to Mihalik describes an anthelmintic paste formulations wherein the avermectin or milbemycin is dissolved in a non-aqueous liquid and pyrantel or morantel, compounds which are in the same class as praziquantel, but are said in the are to be far less effective as praziquantel, are suspended in the liquid. These prior patents do not describe a formulation wherein both the praziquantel and the avermectin or milbemycin that are in a chewable formulation. For example, U.S. Patent 6,165,987 describes anthelmintic formulations containing praziquantel and at least one avermectin or milbemycin dissolved in an ester or ester-like compounds, such as glycerol formal, benzyl alcohol and N-methyl-2-pyrrolidone, which may be liquids, pastes or drenches no mention is made of a chewable formulation or one which is both non-animal products containing and a palatable to the animal.

The term "pharmaceutical agent" or "therapeutic agent" also includes the pharmaceutically or veterinary acceptable acid or base salts, where applicable, of these compounds. The term "acid" contemplates all pharmaceutically or veterinary acceptable inorganic or organic acids. Inorganic acids include mineral acids such as hydrohalic acids, such as hydrobromic and hydrochloric acids, sulfuric acids, phosphoric acids and nitric acids. Organic acids include all pharmaceutically or veterinary acceptable aliphatic, alicyclic and aromatic carboxylic acids, dicarboxylic acids tricarboxylic acids and fatty acids. Preferred acids are straight chain or branched, saturated or unsaturated C₁-C₂₀ aliphatic carboxylic acids, which are optionally substituted by halogen or by hydroxyl groups, or C₆-C₁₂ aromatic carboxylic acids. Examples of such acids are carbonic acid, formic acid, fumaric acid, acetic acid, propionic acid, isopropionic acid, valeric acid, α-hydroxy acids, such as glycolic acid and lactic acid, chloroacetic acid, benzoic acid, methane sulfonic acid, and salicylic acid. Examples of dicarboxylic acids include oxalic acid, malic acid, succinic acid, tataric acid and maleic acid. An example of a tricarboxylic acid is citric acid. Fatty acids include all pharmaceutically or veterinary acceptable saturated or unsaturated aliphatic or aromatic carboxylic acids having 4 to 24 carbon atoms. Examples include butyric acid, isobutyric acid, sec-butyric acid, lauric acid, palmitic acid, stearic acid, oleic acid, linoleic acid, linolenic acid, and phenylsteric acid. Other acids include gluconic acid, glycoheptonic acid and lactobionic acid.

The term "base" contemplates all pharmaceutically or veterinary acceptable inorganic or organic bases. Such bases include, for example, the alkali metal and alkaline earth metal salts, such as the lithium, sodium, potassium, magnesium or calcium salts. Organic bases include the common hydrocarbyl and heterocyclic amine salts, which include, for example, the morpholine and piperidine salts.

The ester and amide derivatives of these compounds, where applicable, are also contemplated. Specific compounds which belong to these classes of therapeutic agents are well known to the practitioner of this art.

Other pharmaceutical agents, such as vitamins, mineral supplements, which are know in the veterinary art are also contemplated.

An important feature of the present invention is the flavor that does not contain animal products or is not derived from an animal source. Flavors derived from catnip, the valarian plant or fruit are not contemplated by the present invention. Flavors include those know in pet foods which are artificial and include, for example:

| DRY GARLIC-ADE OS | Formulated to provide a pungent garlic aroma. |
|---|---|
| LIQUID GARLIC-ADE OS | Same as dry garlic-ade in an oil miscible liquid form. |
| LIQUID GARLIC-ADE CONCENTRATE OM | Same as Dry Garlic-Ade but in a concentrated, oil miscible liquid form. |
| DRY ONION-ADE | Formulated to deliver an aroma a nd taste of cooked onions. |
| DRY GARLIC ONION-ADE | A dry blend of Garlic-Ade and Onion-Ade. |
| DRY CHEESE-ADE | A strong cheddar cheese flavor and aroma. |
| LIQUID CHEESE-ADE OM | An oil miscible, liquid version of Dry Cheese-Ade. |
| DRY CHICKEN-ADE | Formulated to provide the aroma of baked chicken. |
| LIQUID CHICKEN-ADE OS | An oil soluble liquid version of Dry Chicken-Ade. |
| LIQUID CHICKEN-ADE OS CONCENTRATE FFA | A concentrated form of Liquid Chicken-Ade OS. |
| DRY LIVER-ADE | Formulated to provide the aroma and flavor of cooked liver. |
| LIQUID LIVER-ADE CONCENTRATE | A concentrated liquid version of Dry Liver-Ade. |
| DRY PET-ADE BEEF STEW | A blend of many flavor components which provide of beef stew. |
| LIQUID PET-ADE BEEF STEW OS | An oil soluble, liquid version of Dry Pet-Ade Beef Stew. |
| PET-ADE BEEF STEW CONCENTRATE | A concentrated liquid version of Dry Pet-Ade Beef Stew. |
| DRY BEEF-ADE | A dry flavor formulated to provide the appeal of a baking roast. |
| DRY FISH MEAL FLAVOR CONCENTRATE | A dry flavor formulated to provide the odor of fish meal. |
| LIQUID FISH MEAL FLAVOR CONCENTRATE | A liquid version of Dry Fish Meal Flavor. |
| DRY KANIN-KRAVE | A spicy bone marrow flavor. |
| DRY BACON-ADE | A dry flavor which provides the aroma of frying bacon. |

Sources for these flavors are well-know to a practitioner in this art. For example, Kermine Petfood Nutrisurance is a vegetarian flavor for pet food is sold by Kemine industries, Inc., Des Moines, IW. A discussion of commercial smoke flavorings is provided by Guillen et al. in J. Agr. and Food Chemistry vol. 4.

Preferred are the GRILLIN' line of grill flavors and blends marketed by the Red Arrow Products Company, LLC, Manitowoc, WI for human and pet food. These include GRILLIN' TYPE CB-200, GRILLIN' TYPE SD, GRILLIN' TYPE WS-50, GRILLIN' TYPE CN, GRILLIN'TYPE CB, GRILLIN' TYPE GS and GRILLIN' TYPE NBF.

Especially preferred are hickory smoked flavoring produced by combining torula yeast and an aqueous hickory smoke solution, sold by Red Arrow Products Co. as CHARTOR HICKORY or a hickory smoke flavoring produced by combining maltodextin with an aqueous hickory smoke solution, sold by Red Arrow Products Co. as CHARDEX HICKORY. Other flavors contemplated by the invention include those which impart a natural dry smoke flavor. These include CHARZYME (a smoke flavor produced by combining barley malt flour with an aqueous smoke flavor), CHARMAIZE (a smoke flavor produced by combining yellow flower and an aqueous smoke flavor) and CHARSALT (a blend of dendritic salt, aqueous smoke flavor, and dydrated silicon dioxide. All of these flavors may be obtained by Red Arrow Products Co.

The determination of the amounts of flavor for a particular product is easily determined by a practitioner of this art. Typical ranges are from up to about 10%. Also preferred are those flavors which provide a savory flavor. These flavors are well known to a practitioner of this art.

Absorbents may also be added to the inventive formulations. Such compounds are well known in the art to the practitioner as well as their use in pastes. These compounds effectively prevents or alleviates the phase separation of the product during storage. Preferred absorbents include magnesium carbonate, calcium carbonate, potassium bicarbonate, sodium bicarbonate, starch, cellulose and its derivatives, or mixtures of absorbents, with magnesium carbonate being especially preferred. The inclusion of these compounds is optional with amounts of 0% to about 30%, 0 to about 15% or about 1% to about 15% or about 1% to about 10%, based on total weight of the formulation being especially preferred.

Additionally, the disclosed formulations may contain other inert ingredients such as antioxidants, preservatives, stabilizers or surfactants. These compounds are well known in the formulation art. Antioxidant such as an alpha tocopheral, ascorbic acid, ascrobyl palmitate, fumeric acid, malic acid, sodium ascorbate, sodium metabisulfate, n-propyl gallate, BHA (butylated hydroxy anisole), BHT (butylated hydroxy toluene) monothioglycerol and the like, may be added to the present formulation. The antioxidants are generally added to the formulation in amounts of from about 0.01 to about 2.0%, based upon total weight of the formulation, with about 0.1 to about 1.0% being especially preferred. Preservatives, such as the parabens (methylparaben and/or propylparaben), are suitably used in the formulation in amounts ranging from about 0.01 to about 2.0%, with about 0.05 to about 1.0% being especially preferred. Other preservatives include benzalkonium chloride, benzethonium chloride, benzoic acid, benzyl alcohol, bronopol, butylparaben, cetrimide, chlorhexidine, chlorobutanol, chlorocresol, cresol, ethylparaben, imidurea, methylparaben, phenol, phenoxyethanol, phenylethyl alcohol, phenylmercuric acetate, phenylmercuric borate, phenylmercuric nitrate, potassium sorbate, sodium benzoate, sodium propionate, sorbic acid, thimerosal, propyl paraben, myristyl gama-picolinium chloride, paraben methyl, paraben propyl and quaternary ammonium compounds and the like.

Surfactants in amounts from a bout 0.001 to a bout 1 %, based upon total weight may also be added to help solubilize the active drug, to prevent crystallization, and to prevent phase separation. Some examples of the surfactants are: glyceryl monooleate, polyoxyethylene sorbitan fatty acid esters, sorbitan esters, polyvinyl alcohol, P luronics, polysorbate 80, sodium lauryl sulfate, poloxomers (LUTROL F87), etc. Again, these compounds, as well as their amounts are well known in the art.

Colorants may be added to the inventive formulations. Colorants contemplated by the present invention are those commonly known in the art. Specific colorants include, for example, dyes, an aluminum lake, caramel (which may also function as a flavor), colorant based upon iron oxide or a mixture of any of the foregoing. Especially preferred are organic dyes and titanium dioxide. Preferred ranges include from about 0.5% to about 25%.

The chewable formulations provided for in the invention may also include lubricants, such as polyethylene glycols (PEG's or CARBOWAX), corn oil, mineral oil, hydrogenated vegetable oils (STEROTEX OR LUBRITAB), peanut oil and/or castor oil. The inclusion and identity of a lubricant is readily determined by a practitioner of this art are present in amounts, for example, of about 0.01 to about 20%, based upon total weight in the composition.

Compounds which stabilize the pH of the formulation (pH modifiers) are also contemplated. Again, such compounds are well known to a practitioner in the art as well as how to use these compounds. Buffering systems include, for example, systems selected from the group consisting of acetic acid/acetate, malic acid/malate, citric acid/citrate, tataric acid/tartrate, lactic acid/lactate, phosphoric acid/phosphate, glycine/glycimate, tris, glutamic acid/glutamates and sodium carbonate. Preferred ranges for pH include from about 4 to about 6.5.

Other compounds contemplated by the inventive formulations include complexing agents, such as cyclodextrins, PVP, PEG, ethyl lactate and niacinamide. Amounts of such compounds to be included in the inventive formulation are well known to a practitioner of the art. Also contemplated are therapeutic agents to be in the form of emulsions, liposomes or micelles

The inventive formulation may be administered to a warm-blooded animals, such as cattle, sheep, pigs, cats, dogs, horses, llamas, deer, rabbits, skunks, raccoons, camels and the like, or birds. The formulations contemplated by the invention can also be used with humans. The amount of pharmaceutical agent depends on the individual therapeutic agent, the animal being treated, the disease state, and the severity of the disease state. The determination of those factors is well within the skill level of the practitioner. Generally, such preparation normally contain about 0.0005 to about 50% of therapeutic agent by total weight of composition. Preferred formulations are those containing about 0.01 to 10% of therapeutic agent and especially preferred formulations are those containing about 2.5 to about 5% of therapeutic agent. Other preferred amounts include about 0.1 to about 0.01 to about 50% or about 10% or about 0.5 to about 3%. For the avermectins and milbemycins, the formulations will generally be prepared to administer from about 0.1 to about 2 mg/kg, preferably from about 0.4 to about 0.85 mg/kg and most preferably from about 0.6 to about 0.7 mg/kg of the active ingredient. At a preferred dose volume of about 1 ml to treat 50 kg of animal body weight the formulation contains from about 5 to about 50 mg of the active agent per ml of solution or about 0.5 to about 10%, preferably about 2.5 to about 5% w/v. However, depending upon the activity of the compound and the animal being treated, doses as low as about 0.3% of the active ingredient are usable. For nodulisporic acid and its derivatives, a formulation containing about 0.0005 to about 5% of the active compound is preferred.

For chewable veterinary formulation comprising an avermectin or a milbemycin and an antiparasitic agent for nematodes or trematodes, such as praziquantel or pyrantel, preferred amounts of praziquantel include, for example, from about 0.5 mg/kg to about 7.5 mg/kg of animal body weight, with a range of about 0.5 mg/kg to about 2 mg/kg or 2.5 mg/kg of body weight being especially preferred. A most especially preferred a mount is a bout 1.0 mg/kg of animal body weight. P referred ranges for the anthelmintic macrolide compounds include, for example about 0.01 to about 200 mg/kg of animal body weight, with the ranges of about 0.1 to about 50 mg/kg and from about 1 to about 30 mg/kg being especially preferred.

This disclosure further provides for tablets that do not contain animal products which comprise, in addition to the non-animal product containing flavor or flavor derived from a non-animal source, at least one pharmaceutical agent, flavor, filler, lubricant, and flow aid. Optionally, the disclosed tablets may further contain at least one of the following ingredients: colorants, binders, antioxidants, disintegrants, or preservatives. Moreover, in an alternative embodiment this disclosure provides for tablets which are coated. The tablets are prepared according to methods conventional in the art, such as wet and dry granulation processes.

Many of the ingredients for the tablet include those provided for in the chewable formulations. With respect to fillers (or diluents), the disclosed tablets contemplate all the fillers which are known in the tablet art. Non-limiting examples of fillers include anhydrous lactose, hydrated lactose, sprayed dried lactose, crystalline maltose and maltodextrins.

Flow aids or glidants are also well known in the art and include, for example, silicon dioxide (CARBOSIL) or silica gel (SYLOID), talc, starch, calcium, stearate, magnesium stearate, and aluminum magnesium silicate (NEUSILIN). Amounts of flow aids are readily determined by a practitioner in this art and include for using about 0.01 to about 25%, based upon weight of total composition. Non-limiting examples of lubricants for the tablets include magnesium and calcium stearate and stearic acid. Again, the various lubricants are well known to a practitioner of this art as well as the amounts of these compounds. Ranges include from about 0.01 to about 20%.

The tablets provided for by this disclosure may be coated using techniques conventional in the art. Coatings include sugar coatings, such as seal coatings, subcoatings, and syrup coatings, as well as film coatings, such as pan-pour coatings and pan spray coatings. As well known to a practitioner of this art, the coatings contain additional components such as solvents, plasticizers, colorants, opaquant-extenders and film formers.

The present disclosure also provides for a process to prepare the inventive chewable veterinary formulations which is easier and less inexpensive than when animal byproducts or flavors derived from animal products are used because a drying step is eliminated. The manufacturing process comprises the following steps:
a) blending the pharmaceutical agent, binder, disintegrant and non-animal product containing flavor or flavor derived from a non-animal source;
b) adding the water and the humectant to the mixture obtain in step (a) and mixing the mixture; and
c) without drying, extruding the mixture.

The inventive oral formulations may be used to treat a number of disease states by administering to the host in need thereof an effective amount of the oral formulation containing the pharmaceutical agent. The determining of a treatment protocol of a specific indication would be well within the skill level of a practitioner in the pharmaceutical or veterinary arts. Disease states which may be treated by the inventive formulations include, for example, treating inflammation, treating osteoarthritis and rheumatoid arthritis pain or fever, treating or preventing insect or parasitic infestations, treating or preventing bacterial infections; or inhibiting excess acid secretions in the stomach for treating stomach ulcers. The hosts include all animals, e.g. cats, dogs, cattle, sheep, horses, and pigs. As mentioned above, the oral formulation provided for by this invention also could be used to treat disease states in human hosts.

### EXAMPLES

A better understanding of the present invention and of its many advantages will be had from the following examples, given by way of illustration.

### Example 1: Palatability Studies

This test determined which of the four alternative, non-animal product containing flavors for a COX-2 inhibitor would be most readily be accepted by dogs in a daily home-use situation. The four alternative, non-animal flavors were selected from a field of sixteen flavors in qualitative testing with employee dogs. The control was a tablet which contained real pork liver.

The formulations, which were in the form of tablets, were prepared as follows:
Control: Formulation containing 6% real pork liver:

| INGREDIENT | MANUFACTURER | % w/w |
|---|---|---|
| Stock Granulation | | 92.9 |
| Natural Liver Flavor | American Laboratories | 6.0 |
| Magnesium Stearate | | 1.1 |
| Lactose | Foremost | 0.0 |
| Total | | 100.0 |

Inventive: Formulation containing 4% CHARDEX

| INGREDIENT | MANUFACTURER | % w/w |
|---|---|---|
| Stock Granulation | | 92.9 |
| CHARDEX | Red Arrow | 4.0 |
| Magnesium Stearate | | 1.1 |
| Lactose | Foremost | 2.0 |
| Total | | 100.0 |

Inventive: Formulation containing 2 % CHARDEX

| INGREDIENT | MANUFACTURER | % w/w |
|---|---|---|
| Stock Granulation | | 92.9 |
| CHARDEX Flavor | Red Arrow | 2.0 |
| Magnesium Stearate | | 1.1 |
| Lactose | Foremost | 4.0 |
| Total | | 100.0 |

Inventive: Formulation containing 4% CHARTOR

| INGREDIENT | MANUFACTURER | % w/w |
|---|---|---|
| Stock Granulation | | 92.9 |
| CHARTOR Flavor | Red Arrow | 4.0 |
| Magnesium Stearate | | 1.1 |
| Lactose | Foremost | 2.0 |
| Total | | 100.0 |

Inventive: Formulation containing 2% CHARDEX and 2% Carmel

| INGREDIENT | MANUFACTURER | % w/w |
|---|---|---|
| Stock Granulation | | 92.9 |
| CHARDEX | Red Arrow | 2.0 |
| Carmel | Foote & Jenks | 4.0 |
| Magnesium Stearate | | 1.4 |
| Total | | 100.0 |

The stock granulation contained the following ingredients:

| INGREDIENT | MANUFACTURER | % w/w |
|---|---|---|
| Open Flavor (added later) | FMC | 6.0 |
| Avicel PH 102 | FMC | 15.0 |
| AcDiSol | FMC | 2.8 |
| Magnesium Stearate (added later) | | 1.1 |
| Cab O Sil | Cabot | 0.6 |
| Klucel EXF | Hercules | 3.0 |
| Yellow Iron Oxide | Colorcon | 0.13 |
| Red Iron Oxide | Colorcon | 0.27 |
| Fast Flo Lactose | Foremost | 71.1 |
| Total | | 100.00 |

The results of the trials are summarized below:

**TABLE I: Paletability Study**

| | | | | | | Chardex |
|---|---|---|---|---|---|---|
| | | Pork Liver | Chardex 4% | Chardex 2% | Chartor 4% | 2% |
| (N = Total Dogs That Tried Flavor) | | (98) | (85) | (94) | (83) | +Carmel |
| | | | | | | 4% |
| | | | | | | (79) |
| *Mean Days 1-5* | | % | % | % | % | % |
| Accepted tablet (net) | | 94 | 74 | 74 | 85 | 79 |
| Accepted plain tablet 1^{st} attempt | 5 | 80 | 26 | 32 | 60 | 38 |
| Accepted plain tablet > 1 attempt | 4 | 10 | 10 | 14 | 13 | 18 |
| Accepted tablet with food/treat 1^{st} attempt | 3 | 3 | 23 | 13 | 9 | 11 |
| Accepted tablet with food/treat >1 attempt | 2 | 1 | 12 | 13 | 2 | 8 |
| | | 83 | 49 | 45 | 68 | 49 |
| Accepted 1^{st} attempt (subnet) | | | | | | |
| Accepted > 1 attempt (subnet) | | | | | | |
| Accepted plain (subnet) | | 11 | 22 | 27 | 15 | 26 |
| Accepted with food/treat (subnet) | | 90 | 36 | 46 | 73 | 57 |
| Did not accept this tablet | | 4 | 35 | 26 | 11 | 19 |
| Mean | | 6 | 26 | 26 | 15 | 21 |
| | | 4.6 | 2.9 | 3.1 | 4.0 | 3.4 |

The four synthetic test flavors were accepted by the dogs although not as readily as the formulations flavored with real pork liver.
- Specifically, 94% of the dogs accepted the Pork liver tablets overall, with 80% accepting it plain on the first attempt (refusal rate was 6%).
- For the artificial flavors, 74% to 85% of the dogs accepted the tables overall, with a range of 25% to 60% of these accepting the tablets plain on the first attempt (i.e., refusal rates were 15% to 26%).
- Pork liver scores of "Accepted - 94%," "Accepted plain, 1^{st} attempt - 80%," "Accepted plain - 90%," and "Accepted 1^{st} attempt - 83%" are significantly higher than scores for all other tablets at 95% + level of confidence.
- CHARTOR was accepted by 85% of the dogs compared to 74-79% for the CHARDEX options. CHARTOR also was more readily accepted, with 60% accepting the tablet plain on the first attempt compared to 26 to 38% for the CHARDEX options.
- Overall "Accepted" score significantly higher than CHARDEX 2 % and 4 % options at 90% + level of confidence.
- There were no statistically significant differences between CHARTOR and CHARDEX + Carmel.
- There were no statistically meaningful differences in scores between the CHARDEX 2%, 4% and +Caramel options.

While dog owners considered the synthetically flavored formulations more difficult to administer, the "easy" score for formulations flavored with CHARTOR were very acceptable.

**TABLE II: Ease of Administration**

| | | Pork Liver | CHARDEX | CHARDEX | CHARTOR | CHARDEX 2% |
|---|---|---|---|---|---|---|
| (N = Total Dogs That Tried Flavor) | | 6% | 4% | 2% | 4% | ± |
| | | (98) | (84) | (94) | (82) | Carmel 4% (79) |
| *Mean Days 1-5* | | % | % | % | % | % |
| Easy (net) | | 91 | 58 | 61 | 82 | 68 |
| Very easy | 4 | 80 | 34 | 40 | 69 | 46 |
| Somewhat easy | 3 | 11 | 24 | 21 | 13 | 22 |
| | | | | | | |
| Somewhat difficult | 2 | 3 | 19 | 18 | 9 | 12 |
| Very difficult | 1 | 6 | 20 | 20 | 9 | 18 |
| Difficult (net) | | 9 | 40 | 37 | 18 | 30 |
| | | | | | | |
| Mean | | 3.7 | 2.7 | 2.8 | 3.4 | 2.9 |

### Example 2: Study to Determine the Acceptability of Place Non-beef Chewable Formulations in Dogs:

**Table III: Animal descriptions and sequences**

| Animal ID | Sex | Age (months) | Sequence # | Formulation # Day 0 | Formulation # Day 2 | Formulation # Day 4 |
|---|---|---|---|---|---|---|
| 1 | F | 68.2 | 1 | 1 | 2 | 3 |
| 2 | M | 46.1 | 2 | 1 | 3 | 2 |
| 3 | F | 39.5 | 3 | 2 | 1 | 3 |
| 4 | F | 17.9 | 4 | 2 | 3 | 1 |
| 5 | M | 33.9 | 5 | 3 | 1 | 2 |
| 6 | M | 18.9 | 6 | 3 | 2 | 1 |

| | | | | | | |
|---|---|---|---|---|---|---|
| M = Male, F = Female | | | | | | |

All dogs were Beagles and originally acquired from either Harlan Sprague Dawley, Madison, WI, Merial Limited, Athens, GA, or Sinclair Research Center, Columbia, MO.

**Formulation 1:**

| | |
|---|---|
| Soy Protein Fines | 45% |
| Explotab | 22% |
| CHARDEX Flavor | 3% |
| Povidone K=90 | 4% |
| Water | 20% |
| Propylene Glycol | 6% |

**Formulation 2:**

| | |
|---|---|
| Soy Protein Fines | 47% |
| Explotab | 22% |
| CHARDEX Flavor | 1% |
| Povidone K=90 | 4% |
| Water | 20% |
| Propylene Glycol | 6% |

**Formulation 3:**

| | |
|---|---|
| Soy Protein Fines | 47% |
| Explotab | 22% |
| Chocolate Flavor | 1% |
| Povidone K=90 | 4% |
| Water | 20% |
| Propylene Glycol | 6% |

All chewables were offered once on either Day 0, 2, or 4.

**Table IV: Acceptability Scores**

| | FORMULATION 1 | FORMULATION 2 |
|---|---|---|
| 1 | 2* | 1 |
| 2 | 1 | 1 |
| 3 | 1 | 1 |
| 4 | 2* | 1 |
| 5 | 1 | 1 |
| 6 | 1 | 2* |

| | | |
|---|---|---|
| All chewables were offered once on either Day 0, 2, or 4. Acceptability Scoring System: 1 = Swallowed readily 2 = Swallowed with coaxing or food 3 = Refused *An acceptability score of 2 was recorded as such because the dog played with the chewable before eating it. No chewable had to be given with food. | | |

### Example 2

A non-beef chewable formulation comprising the following components:

### Eprinomectin-Praziquantel Chewable Formulation 1

| | **Ingredients** | **Source** | **%** |
|---|---|---|---|
| 1. | Polyethylene Glycol 400 | JTBaker | 20 |
| 2. | Tenox 2 | Eastman | 0.02 |
| 3. | Lutrol F87 | BASF | 0.5 |
| 4. | Eprinomectin* | Merck | 0.0114 |
| 5. | Praziquantel** | Merck | 4.25 |
| 6. | Soy Protein Fines*** | ADM | 37.719 |
| 7. | Art. Beef Flavor PC | PC | 15 |
| 8. | Crospovidone | ISP | 5 |
| 9. | Povidone K-90 | ISP | 2 |
| 10. | Citric Acid | NA | 1 |
| 11. | Potassium Sorbate | Spectrum | 0.5 |
| 12. | Purified Water | Merial | 10 |
| 13. | Corn Oil | Sigma | 4 |
| **TOTAL** | | | **100** |

| | | | |
|---|---|---|---|
| *Amount of Eprinomectin on the basis of CoA: 100%/(% Assay) 97.4% x 0.0114 x 2g = 0.023 g **Amount of Praziquantel on the basis of CoA: 100%/(% Assay) 99% x4.25 x 2g = 8.856 g ***Adjust amount of Soy Protein Fines according to the amount of Eprinomectin & Praziquantel: 75.351 g | | | |

This formula was prepared as follows:
1. Mix components 1 and 2.
2. Dissolve with stirring components 3, 4 and 5 in step 1 in sequence. If necessary, use heating to dissolve.
3. Mix items 6 to 9 in a planetary mixer for 10 minutes.
4. Granulate step 3 with solution of step 2.
5. Dissolve Citric Acid in 50% of water and add to step 3.
6. Dissolve Potassium Sorbate in rest of the water and add to step 3.
7. Mix as required.
8. Add Corn Oil & mix.
9. Make extrudate.
10. the extrudates at 50°C for 2 hour.

### Example 3

A non-animal product containing chewable formulation comprising the following components:

### Eprinomectin-Praziquantel Chewable

**Formulation 1**

| # | **Ingredients** | **Source** | **%** |
|---|---|---|---|
| 1. | Propylene Glycol | JTBaker | 20 |
| 2. | Tenox 2 | Eastman | 0.02 |
| 3. | Sod. Lauryl Sulfate | Fisher | 0.5 |
| 4. | Eprinomectin* | Merck | 0.0114 |
| 5. | Praziquantel** | E Merck | 4.25 |
| 6. | Emdex | Penwest | 10 |
| 7. | Pregelatinized Starch | Colorcon | 10 |
| 8. | Corn Starch*** | NA | 21.719 |
| 9. | Art. Beef Flavor PC | Pharma C | 15 |
| 10. | Crospovidone | ISP | 5 |
| 11. | Citric Acid | Sigma | 1 |

| | **Ingredients** | **Source** | **%** |
|---|---|---|---|
| 12. | Potassium Sorbate | Spectrum | 0.5 |
| 13. | Purified Water | Merial | 8 |
| 14. | Corn Oil | Sigma | 4 |
| **TOTAL** | | | **100** |

| | | | |
|---|---|---|---|
| *Amount of Eprinomectin on the basis of CoA: 100%/(% Assay) 97.4% x 0.0114 x 2g = 0.023 g **Amount of Praziquantel on the basis of CoA: 100%/(% Assay) 99% x 4.25 x 2g = 8.586 g ***Adjust amount of Corn Starch according to the amount of Eprinomectin & Praziquantel = 43.351 g. | | | |

The above formula was prepared as follows:
1. Mix items 1 and 2.
2. Dissolve with stirring items 3, 4 and 5 in step 1 in sequence. Heat if necessary.
3. Mix items 6 to 10 in a planetary mixer for 10 minutes.
4. Granulate step 3 with solution of step 2.
5. Mix for 10 minutes or as required.
6. Dissolve citric acid in 8 g of Water. Continue granulation of step 5.
7. Dissolve potassium sorbate in 8 g of water. Add to step 5 & continue granulation.
8. Add Corn Oil. Mix for 5 minutes.
9. Make extrudate.
10. Dry the extrudates at 50°C for 2 hour.

### Example 4

A non-animal product contains tablet formulations comprising the following components is prepared using a conventional tableting technique

**COX-2 Tablet**

| **INGREDIENT** | **CENTESIMAL COMPOSITION (w/w%)** |
|---|---|
| 3-(cyclopropylmethoxy)-5,5-dimethyl-4-(4-methylsulfonyl)phenyl)-5H-furan-2-one or 3-(cyclopropylethoxy)5,5-dimethyl-4-(4-methylsulfonyl)phenyl)-5H-furan-2-one | 24.0 |
| Microcrystalline Cellulose | 15.0 |
| Chartor Hickory | 3.0 |
| Caramel | 1.0 |
| Yellow Iron Oxide | 0.3 |
| Red Iron Oxide | 0.1 |
| Hydroxypropyl Cellulose | 3.0 |
| Croscarmellose Sodium | 2.8 |
| Collodial Silicone Dioxide | 0.5 |
| Magnesium Stearate | 1.0 |
| Lactose Monohydrate | 49.3 |

## Claims

1. A chewable veterinary formulation suitable for use in treating dogs, which chewable veterinary formulation does not contain animal products, and which comprises:
- effective amount of at least one pharmaceutical agent;
- at least one filler;
- at least one disintegrant;
- at least one non-animal product containing flavor or flavor derived from a non-animal source;
- at least one binder;
- at least one humectant;
- at least one granulating solvent; and
- optionally, at least one antioxidant, at least one pH modifier, at least one surfactant,
at least one preservative, at least one lubricant or at least one colorant; and
wherein,
- the filler is selected from the group consisting of soy protein, corn cob, and corn gluten meal;
- the disintegrant is selected from the group consisting of sodium starch glycolate, crospovidone, croscarmellose sodium, starch, microcrystalline cellulose, alginic acid, veegum, bentonite, and pregelatinized starch;
- the binder is selected from the group consisting of polyvinyl pyrrolidone, povidone, starch, pregelatinized starch, methylcellulose, hydroxypropyl cellulose, carboxymethyl cellulose sodium, ethylcellulose, sodium alginate, tragacanth and acacia;
- the humectant is selected from the group consisting of propylene glycol, glycerin, and polyethylene glycol 400;
- the granulating solvent is water or an aqueous sorbitol solution; and
wherein the pharmaceutical agent is a compound selected from the group consisting of an antiparasitic agent, nodulisporic acid, estrogens, progestins, androgens, imidacloprid, phenylpyrazols, and proton pump inhibitors.

2. The chewable veterinary formulation according to claim 1, which further comprises an antioxidant and the antioxidant is an alpha tocopherol, ascorbic acid, ascorbyl palmitate, sodium ascorbate, sodium metabisulfate, n-propyl gallate, butylated hydroxy anisole, butylated hydroxy toluene, monothioglycerol or a mixture of any of the foregoing.

3. The chewable veterinary formulation according to claim 2, which further comprises a colorant and the colorant is a dye, an aluminum lake, caramel, colorant based upon iron oxide or a mixture of any of the foregoing.

4. The chewable veterinary formulation according to claim 3, which further comprises a preservative and the preservative is a compound selected from the group consisting of benzalkonium chloride, benzethonium chloride, benzoic acid, benzyl alcohol, bronopol, butylparaben, centrimide, chlorhexidine, chlorobutanol, chlorocresol, cresol, ethylparaben, imidurea, methylparaben, propylparaben, phenol, phenoxyethanol, phenylethyl alcohol, phenylmercuric acetate, phenylmercuric borate, phenylmercuric nitrate, potassium sorbate, sodium benzoate, sodium propionate, sorbic acid, thimerosal, myristyl gamma- picolinium chloride, quaternary ammonium compounds and a mixture of any of the foregoing.

5. The chewable veterinary formulation according to claim 4, wherein the pharmaceutical agent is selected from the group consisting of avermectins, milbemycins, nodulisporic acid, estrogens, progestins, androgens, imidacloprid, phenylpyrazols, praziquantel, 1,4,5,6-tetrahydro-2-[(2-substituted)vinyl pyrimidine, pyrantel and proton pump inhibitors.

6. The chewable veterinary formulation according to claim 5, which further comprises a surfactant selected from the group consisting of glyceryl monooleate, polyoxyethylene, sorbitan esters, polyvinyl alcohol, sodium lauryl sulfate and poloxamers.

7. The chewable veterinary formulation according to claim 5, which further comprises a lubricant and the lubricant is selected from the group consisting of corn oil, polyethylene glycol, mineral oil, hydrogenated vegetable oil, peanut oil or castor oil.

## Patentansprüche

1. Eine kaubare tierärztliche Formulierung, geeignet zur Verwendung bei der Behandlung von Hunden, wobei die kaubare tierärztliche Formulierung keine Tierprodukte enthält und Folgendes umfasst:
- eine wirksame Menge mindestens eines Wirkstoffs;
- mindestens einen Füllstoff;
- mindestens ein Sprengmittel;
- mindestens ein nicht tierisches Produkt, das Aroma enthält, oder ein Aroma, das aus einer nicht tierischen Quelle abgeleitet ist;
- mindestens ein Bindemittel;
- mindestens ein Feuchthaltemittel;
- mindestens ein granulierendes Lösungsmittel; und
- gegebenenfalls mindestens ein Antioxidationsmittel, mindestens einen pH-Modifikator, mindestens ein oberflächenaktives Mittel, mindestens einen Konservierungsstoff, mindestens ein Gleitmittel oder mindestens ein farbgebendes Mittel; und
wobei,
- der Füllstoff aus der Gruppe bestehend aus Sojaprotein, Maiskolben und Maiskleber ausgewählt ist;
- das Sprengmittel aus der Gruppe bestehend aus Natriumstärkeglycolat, Crospovidon, Croscarmellosenatrium, Stärke, mikrokristalliner Cellulose, Alginsäure, Veegum, Bentonit und Quellstärke ausgewählt ist;
- das Bindemittel aus der Gruppe bestehend aus Polyvinylpyrrolidon, Povidon, Stärke, Quellstärke, Methylcellulose, Hydroxypropylcellulose, Carboxymethylcellulosenatrium, Ethylcellulose, Natriumalginat, Traganth und Akazie ausgewählt ist;
- das Feuchthaltemittel aus der Gruppe bestehend aus Propylenglycol, Glycerin und Polyethylenglycol 400 ausgewählt ist;
- das granulierende Lösungsmittel Wasser oder eine wässrige Sorbitollösung ist; und
wobei der Wirkstoff eine Verbindung, ausgewählt aus der Gruppe bestehend aus einem antiparasitären Mittel, Nodulisporinsäure, Östrogenen, Progestinen, Androgenen, Imidacloprid, Phenylpyrazolen und Protonenpumpenhemmern, ist.

2. Die kaubare tierärztliche Formulierung gemäß Anspruch 1, die ferner ein Antioxidationsmittel umfasst, wobei das Antioxidationsmittel ein alphat-Tocopherol, Ascorbinsäure, Ascorbylpalmitat, Natriumascorbat, Natriummetabisulfat, n-Propylgallat, butyliertes Hydroxyanisol, butyliertes Hydroxytoluol, Monothioglycerin oder ein Gemisch aus beliebigen der vorstehend genannten ist.

3. Die kaubare tierärztliche Formulierung gemäß Anspruch 2, die ferner ein farbgebendes Mittel umfasst, wobei das farbgebende Mittel ein Farbstoff, ein Aluminium-Lake, Karamell, ein Farbstoff auf der Basis von Eisenoxid oder ein Gemisch aus beliebigen der vorstehend genannten ist.

4. Die kaubare tierärztliche Formulierung gemäß Anspruch 3, die ferner einen Konservierungsstoff umfasst, wobei der Konservierungsstoff eine Verbindung, ausgewählt aus der Gruppe bestehend aus Benzalkoniumchlorid, Benzethoniumchlorid, Benzoesäure, Benzylalkohol, Bronopol, Butylparaben, Centrimid, Chlorhexidin, Chlorbutanol, Chlorkresol, Kresol, Ethylparaben, Imidharnstoff, Methylparaben, Propylparaben, Phenol, Phenoxyethanol, Phenylethylalkohol, Phenylquecksilberacetat, Phenylquecksilberborat, Phenylquecksilbernitrat, Kaliumsorbat, Natriumbenzoat, Natriumpropionat, Sorbinsäure, Thimerosal, Myristyl-gamma-Picoliniumchlorid, quaternären Ammoniumverbindungen und einem Gemisch aus beliebigen der vorstehend genannten, ist.

5. Die kaubare tierärztliche Formulierung gemäß Anspruch 4, wobei der Wirkstoff aus der Gruppe bestehend aus Avermectinen, Milbemycinen, Nodulisporinsäure, Östrogenen, Progestinen, Androgenen, Imidacloprid, Phenylpyrazolen, Praziquantel, 1,4,5,6-Tetrahydro-2-[(2-substituiertem)vinylpyrimidin, Pyrantel und Protonenpumpenhemmern ausgewählt ist.

6. Die kaubare tierärztliche Formulierung gemäß Anspruch 5, die ferner ein oberflächenaktives Mittel, ausgewählt aus der Gruppe bestehend aus Glycerinmonooleat, Polyoxyethylen, Sorbitanestern, Polyvinylalkohol, Natriumlaurylsulfat und Poloxameren, umfasst.

7. Die kaubare tierärztliche Formulierung gemäß Anspruch 5, die ferner ein Gleitmittel umfasst, wobei das Gleitmittel aus der Gruppe bestehend aus Maisöl, Polyethylenglycol, Mineralöl, hydriertem Pflanzenöl, Erdnussöl oder Rizinusöl ausgewählt ist.

## Revendications

1. Formulation vétérinaire à mâcher adaptée pour utilisation dans le traitement de chiens, ladite formulation vétérinaire à mâcher ne contenant pas de produits d'origine animale, et qui comprend :
- une quantité efficace d'au moins un agent pharmaceutique ;
- au moins une charge ;
- au moins un délitant ;
- au moins un produit non animal contenant un arôme ou un arôme dérivé d'une source non animale ;
- au moins un liant ;
- au moins un humectant ;
- au moins un solvant de granulation ; et
- facultativement, au moins un antioxydant, au moins un modificateur de pH, au moins un tensioactif, au moins un conservateur, au moins un lubrifiant ou au moins un colorant ; et dans laquelle,
- la charge est choisie dans le groupe constitué de protéine de soja, rafle de maïs, et farine de gluten de maïs ;
- le délitant est choisi dans le groupe constitué des glycolate d'amidon sodique, crospovidone, croscarmellose sodique, amidon, cellulose microcristalline, acide alginique, Veegum, bentonite et amidon prégélatinisé ;
- le liant est choisi dans le groupe constitué des polyvinylpyrrolidone, povidone, amidon, amidon prégélatinisé, méthylcellulose, hydroxypropylcellulose, carboxyméthylcellulose sodique, éthylcellulose, alginate de sodium, adragante et gomme arabique ;
- l'humectant est choisi dans le groupe constitué des propylène glycol, glycérine et polyéthylène glycol 400 ;
- le solvant de granulation est l'eau ou une solution aqueuse de sorbitol ; et
dans laquelle l'agent pharmaceutique est un composé choisi dans le groupe constitué d'un agent antiparasitaire, l'acide nodulisporique, des oestrogènes, des progestines, des androgènes, l'imidacloprid, des phénylpyrazols, et des inhibiteurs de la pompe à protons.

2. Formulation vétérinaire à mâcher selon la revendication 1, qui comprend en outre un antioxydant et l'antioxydant est un alpha-tocophérol, l'acide ascorbique, le palmitate d'ascorbyle, l'ascorbate de sodium, le métabisulfate de sodium, le gallate de n-propyle, l'hydroxyanisole butylé, l'hydroxytoluène butylé, le monothioglycérol ou un mélange de l'un quelconque de ceux-ci.

3. Formulation vétérinaire à mâcher selon la revendication 2, qui comprend en outre un colorant et le colorant est un pigment, une laque d'aluminium, le caramel, un colorant à base d'oxyde de fer ou un mélange de l'un quelconque de ceux-ci.

4. Formulation vétérinaire à mâcher selon la revendication 3, qui comprend en outre un conservateur et le conservateur est un composé choisi dans le groupe constitué des chlorure de benzalkonium, chlorure de benzéthonium, acide benzoïque, alcool benzylique, bronopol, butylparabène, centrimide, chlorhexidine, chlorobutanol, chlorocrésol, crésol, éthylparabène, imidurée, méthylparabène, propylparabène, phénol, phénoxyéthanol, alcool phényléthylique, acétate phénylmercurique, borate phénylmercurique, nitrate phénylmercurique, sorbate de potassium, benzoate de sodium, propionate de sodium, acide sorbique, thimérosal, chlorure de myristyl-gamma-picolinium, composés d'ammonium quaternaire et un mélange de l'un quelconque de ceux-ci.

5. Formulation vétérinaire à mâcher selon la revendication 4, dans laquelle l'agent pharmaceutique est choisi dans le groupe constitué des avermectines, milbémycines, acide nodulisporique, oestrogènes, progestines, androgènes, imidacloprid, phénylpyrazols, praziquantel, 1,4,5,6-tétrahydro-2-[(2-substitué)vinyl-pyrimidine, pyrantel et inhibiteurs de la pompe à protons.

6. Formulation vétérinaire à mâcher selon la revendication 5, qui comprend en outre un tensioactif choisi dans le groupe constitué des monooléate de glycéryle, polyoxyéthylène, esters de sorbitan" alcool polyvinylique, lamylsulfate de sodium et poloxamères.

7. Formulation vétérinaire à mâcher selon la revendication 5, qui comprend en outre un lubrifiant et le lubrifiant est choisi dans le groupe constitué des huiles de maïs, polyéthylène glycol, huile minérale, huile végétale hydrogénée, huile d'arachide ou huile de ricin.
